# EUROPÄISCHE PATENTSCHRIFT

(11) **EP 0 894 094 B1**
(45) Veröffentlichungstag und Bekanntmachung des Hinweises auf die Patenterteilung: **11.05.2005**
(21) Anmeldenummer: 97904976.4
(22) Anmeldetag: 11.03.1997
(51) Int. Cl.: C07K 14/475, C07K 1/14, A61K 38/18, A61K 7/26, A23K 1/16

(54) **VERFAHREN ZUR GEWINNUNG EINES KOMPLEXES AUS WACHSTUMSFAKTOREN**
PROCESS FOR EXTRACTION OF A GROWTH FACTOR COMPLEX
PROCEDE POUR L'OBTENTION D'UM COMPLEXE DE FACTEURS DE CROISSANCE

(30) Priorität: 15.03.1996 CH 68696; 13.07.1996 EP 96111328
(43) Veröffentlichungstag der Anmeldung: 03.02.1999
(73) Patentinhaber: BioEqual AG, 4132 Muttenz (CH)
(72) Erfinder: MEYER, Hans, CH-4125 Riehen (CH); WASMER, Hermann, CH-4125 Riehen (CH); HOFMANN, Dieter, CH-4153 Reinach (CH)
(86) Internationale Anmeldenummer: PCT/CH1997/000097
(87) Internationale Veröffentlichungsnummer: WO 1997/034929

(56) Entgegenhaltungen:
- EP-A- 0 313 515
- WO-A-92/00994
- WO-A-95/29933
- Y.SHING ET AL.: "PURIFICATION OF POLYPEPTIDE GROWTH FACTORS FROM MILK (IN METHODS OF ENZYMOLOGY, VOL. 146 "PEPTIDE GROWTH FACTORS" PART A)" 1987 , ACADEMIC PRESS, INC. , NEW YORK, N.Y., US XP002032362 siehe Seite 42 - Seite 48

## Beschreibung

Das erfindungsgemässe Verfahren zur Gewinnung eines Komplexes aus Wachstumsfaktoren (Growth Factor Complex, GFC) ist dadurch gekennzeichnet, dass man naturliches und Wachstumsfaktoren-enthaltendes Material mit Wasser versetzt, den pH-Wert durch Zugabe von Säure auf 2.5 bis 3.2 einstellt, die entstandene Fällung vom Überstand abtrennt und den Growth Factor Complex aus der wässrigen Losung durch Zugabe eines mit Wasser mischbaren organischen Losungsmittels ausgewählt aus niederen Alkanolen oder niederen Dialkylketonen, ausfällt, von der flussigen Phase abtrennt und trocknet und das erhaltene Pulver gewünschtenfalls dialysiert

Als natürliches und Wachstumsfaktoren-enthaltendes Material (in der Folge als Ausgangsmaterial bezeichnet) können zum Beispiel Milch von Saugetieren, wie menschliche Milch, Kuhmilch, Ziegenmilch, Schafsmilch, Stutenmilch und dgl., sowie die entsprechende Molke, Vogeleier, wie Huhnereier, Enteneier, Strausseneier und dgl , Fischrogen, wie Forellenrogen, Storrogen und dgl., Blut von Säugetieren, wie menschliches Blut, Rinderblut, Ziegenblut, Schafsblut, Pferdeblut und dgl., Urin von Saugetieren, wie menschlicher Urin, Rinderurin, Ziegenurin, Schafsurin, Pferdeurin und dgl , sowie Bienenhonig, pflanzliche Samen usw. verwendet werden.

Bevorzugte Ausgangsmaterialien sind menschliche Milch, Kuhmilch, Hühnereier, Forellenrogen und Rinderblut. Ein besonders bevorzugtes Ausgangsmaterial ist entfettete Kuhmilch oder die bei der Käseherstellung anfallende Molke bzw. das entsprechende im Handel erhältliche Molkepulver, das beispielsweise mittels Sprühtrocknungsverfahren hergestellt werden kann

Das erfindungsgemässe Verfahren zur Gewinnung des Growth Factor Complex ist auf alle aufgeführten Beispiele für Ausgangsmaterialien anwendbar, generell sogar auf alle Ausgangsmaterialien, die zum Wachstum beitragen oder selbst wachsen müssen (z.B. Samen). Ganz generell kann das erfindungsgemässe Verfahren auf jedes peptid- oder proteinhaltige Ausgangmaterial angewendet werden. Die Wachstumsfaktoren sind Polypeptide und/oder Proteine von besonderem Interesse, da sie spezielle biologische Eigenschaften besitzen. Auf Grund dieser speziellen Eigenschaften eignen sich die Wachstumsfaktoren per se oder insbesondere im Komplex, d.h wenn mehrere kombiniert werden, als Arzneimittel.

Ausgangsmaterialien, die nicht in flüssiger Form vorliegen, werden vor der Bearbeitung mit Wasser versetzt und in eine homogene Lösung gebracht. Fetthaltige Ausgangsmaterialien müssen vor der Bearbeitung in an sich bekannter Weise, beispielsweise durch Entrahmen, entfettet werden.

Die erfindungsgemasse Gewinnung des Growth Factor Complex wird nachstehend ausführlich beschrieben, wobei beispielhaft als Ausgangsmaterial eine entfettete Milch verwendet wird, deren Gehalt an fester Milchtrockensubstanz höher ist als der von natürlicher Milch. Solche Ausgangsmaterialien mit höherem Gehalt an fester Milchtrockensubstanz können hergestellt werden, indem man die natürliche Milch nach der Entfettung durch Abdampfen von Wasser unter schonenden Bedingungen konzentriert oder indem man aus entfetteter Milch gewonnenes Milchpulver (Trockenmilch) mit der geeigneten Menge Wasser vermischt. Vorzugsweise wird als Ausgangsmaterial entfettete Milch verwendet, deren Gehalt an trockener Mitchsubstanz mindestens 100 g/l beträgt. Ein bevorzugtes Ausgangsmaterial ist aus entfetteter Kuhmilch gewonnenes Milchpulver.

Als Säuren, die zur Einstellung des pH-Wertes der das Ausgangsmaterial enthaltenden Lösung auf pH 2,5 bis 3,2 verwendet werden können, kommen allgemein nicht-oxidierende Mineralsäuren und organische Säuren, wie Mono-, Di- und Tricarbonsäuren sowie davon abgeleitete Hydroxysäuren in Betracht. Geeignete Mineralsäuren sind Salzsäure, Schwefelsäure und Phosphorsäure. Geeignete organische Säuren sind Ameisensäure, Essigsäure, Propionsäure, Buttersäure, Oxalsäure, Malonsäure, Bernsteinsäure, Glykolsäure, Milchsäure (α-Hydroxypropionsäure), β-Hydroxypropionsäure, Apfelsäure, Weinsäure, Ascorbinsäure und Zitronensäure. Die Säuren werden vorteilhaft in Form ihrer wässerigen Lösungen eingesetzt. Bevorzugte Säuren sind Ameisensäure, Essigsäure, Milchsäure und Zitronensäure. Besonders bevorzugt ist 80 %-ige Milchsäure. Die Zugabe der Säure erfolgt vorteilhaft in der Weise, dass man die zur Einstellung des pH-Wertes erforderliche Menge der jeweiligen Säure in Form einer wasserigen Losung bei Raumtemperatur unter starkem Rühren in die Milch einfliessen lasst, wobei die Zugabe in der Regel ohne exotherme Reaktion ablauft Vorzugsweise wird durch die Zugabe der Saure ein pH-Wert von 2,5 bis 2.7 eingestellt. Nach der Zugabe der Säure wird die Mischung vorteilhaft 15 bis 30 Minuten nachgerührt.

Die Abtrennung der durch die Zugabe von Säure entstandenen Fallung kann durch Filtration, beispielsweise durch ein engmaschiges Drahtnetz oder durch ein Filtertuch oder durch Zentrifugieren, erfolgen. Es ist vorteilhaft, die Fallung nach dem Filtrieren durch Abpressen von anhaftender Wachstumsfaktoren-enthaltender wässriger Lösung zu befreien Man erhalt eine feinteilige klebrige Fällung. Es ist weiterhin vorteilhaft, die vereinigten Filtrate nochmals zu filtrieren, z.B. durch ein Saft- oder Faltenfilter, um eine vollständige Abtrennung der mit Säure entstandenen Fallung von Überstand zu gewährleisten Die nach Abtrennung der durch Zugabe von Saure entstandenen Fallung erhaltene Wachstumsfaktoren-enthaltende wassrige Lösung ist ein wertvolles Zwischenprodukt und ebenfalls Gegenstand der vorliegenden Erfindung.

Als mit Wasser mischbare Lösungsmittel, die zur Abscheidung des Growth Factor Complex aus der nach Abtrennung der Fällung erhaltenen wassrigen Lösung geeignet sind, kommen niedere Alkanole, wie Methanol, Ethanol, n-Propanol und Isopropanol, sowe niedere Ketone, wie Aceton, Methylethylketon und Diethylketon in Betracht. Bevorzugte mit Wasser mischbare Lösungsmittel sind Isopropanol und Aceton. Besonders bevorzugt ist Aceton.

Die vereinigten Filtrate werden vor der Zugabe eines mit Wasser mischbaren organischen Losungsmittels zur Abscheidung des Growth Factor Complex auf eine Temperatur von 0°C bis 10°C, vorzugsweise 3°C bis 5°C, abgekühlt. Für die Abscheidung des Growth Factor Complex wird unter Rühren das Filtrat mit dem organischen, mit Wasser mischbaren Lösungsmittel in der Regel so versetzt, dass man das organische Lösungsmittel und das Filtrat in einem Volumenverhältnis von 2 : 1 bis 10 : 1 verwendet. Vorzugsweise beträgt das Volumenverhältnis von organischem, mit Wasser mischbarem Lösungsmittel zum Filtrat 4 : 1 bis 6 : 1. Der abgeschiedene Growth Factor Complex kann durch Filtrieren oder durch Zentrifugieren abgetrennt werden. Es wird vorteilhaft mit dem zur Abscheidung verwendeten Lösungsmittel gewaschen und an der Luft oder bei mässig erhöhter Temperatur im Vakuum getrocknet.

Anschliessend kann das erhaltene Pulver zur Aufkonzentration, erwunschtenfalls gegen fliessendes, ionenfreies Wasser, dialysiert werden, wobei die Durchlässigkeit des Diaphragmas so gewählt wird, dass nur Moleküle mit einem Molekulargewicht von weniger als 3,5 kDa durchgelassen werden.

Es wurde gefunden, dass die biologische Aktivität des erhaltenen Produktes gesteigert werden kann, wenn man es nach dem Trocknen mit einem fettlösenden Lösungsmittel extrahiert. Diese Extraktion kann kontinuierlich oder diskontinuierlich in bekannter Weise mit üblichen Apparaturen durchgeführt werden. Die kontinuierliche Extraktion kann beispielsweise in einer Soxhlet-Apparatur durchgeführt werden. Die diskontinuierliche Extraktion wird vorteilhaft in der Weise durchgeführt, dass man das nach dem erfindungsgemässen Verfahren erhaltene Produkt nach dem Trocknen im Lösungsmittel suspendiert, die Suspension einige Minuten, beispielsweise 5 bis 20 Minuten, rührt und dann das Produkt abtrennt und trocknet. Als fettlösende Lösungsmittel kommen insbesondere niedrig siedende halogenierte aliphatische Kohlenwasserstoffe, wie Methylenchlorid, Chloroform und Kohlenstofftetrachlorid, in Betracht. Ein bevorzugtes Lösungsmittel ist Methylenchlorid.

Der nach dem erfindungsgemässen Verfahren gewinnbare Growth Factor Complex aus Kuhmilch ist ein weisses kristallines Pulver. Die in diesem Pulver enthaltenen aktiven Polypeptide, die sogenannten Wachstumsfaktoren, sind teilweise bereits aus der Literatur bekannt (M.R. Pittelkow, Advances in Dermatology, 1991, 7, 55). Die bekanntesten und am besten untersuchten Milch-Wachstumsfaktoren sind:
- Epidermal Growth Factor (EGF)
- Transforming Growth Factor α (TGF-α)
- Transforming Growth Factor β (TGF-β)
- Fibroblast Growth Factor (FGF)
- Platelet Derived Growth Factor (PDGF)

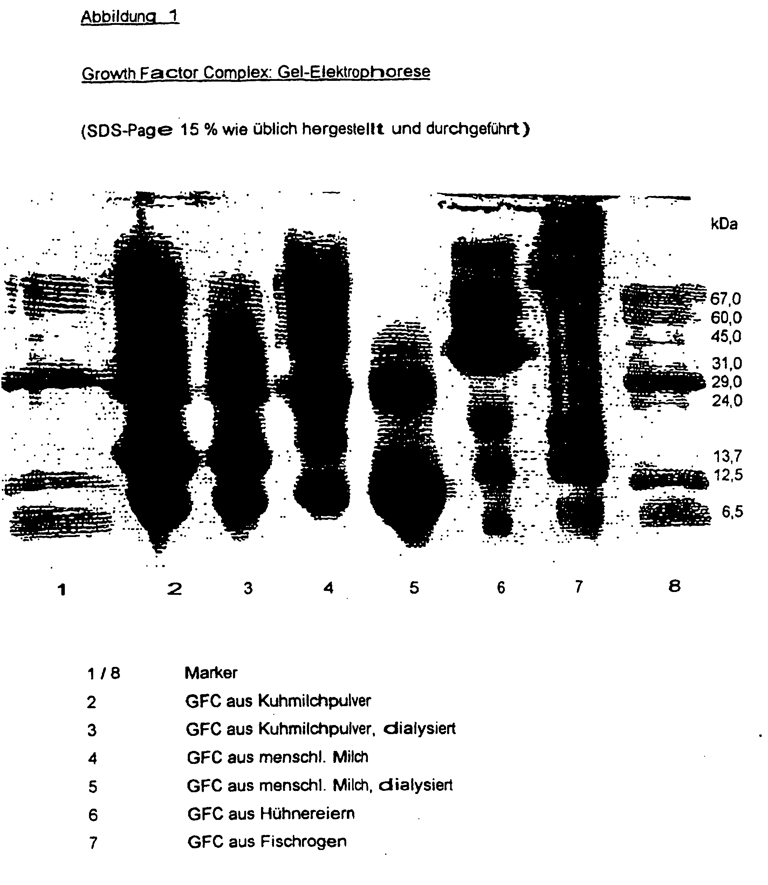

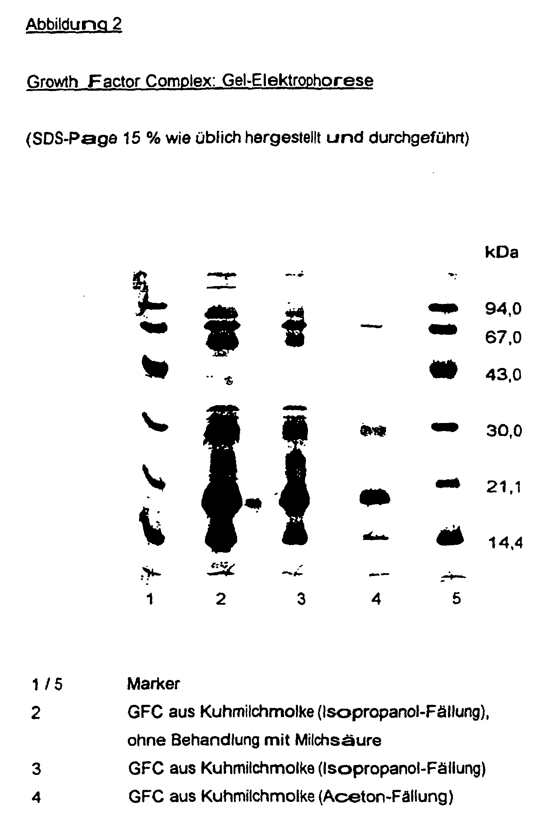

Das erfindungsgemässe Verfahren zur Isolation von Wachstumsfaktoren kann an allen vorgängig beschriebenen Ausgangmaterialien durchgeführt werden. Der resultierende Growth Factor Complex setzt sich allerdings je nach Ausgangsmaterial aus unterschiedlichen aktiven Polypeptiden zusammen. Zu den erfindungsgemäss erhaltenen, verschiedenen Wachstumsfaktoren sind in Tabelle 1 die Massenspektren, in Tabelle 2 die Retentionszeiten und in den Abbildungen 1 und 2 die SDS-Page Gel-Elektrophorese-Diagramme aufgeführt.

**Tabelle 1**

| Growth Factor Complex: Massenspektren (Molgewichte in Dalton) | | | | | | |
|---|---|---|---|---|---|---|
| GFC aus Kuhmilch, dialysiert | GFC aus menschl. Milch, dialysiert | GFC aus Stutenmilch, dialysiert | GFC aus menschl. Blut, dialysiert | GFC aus Molke, dialysiert | GFC aus Hühnereiern, dialysiert | GFC aus Fischrogen, dialysiert |
| | | | | | | 1'744,0 |
| | | | | | | 1'901,0 |
| | | | | | | 2'117,0 |
| | | | | | | 2'263,0 |
| | | | | | | 2'345,0 |
| | | | | | | 2'424,0 |
| | | | | | | 2'516,0 |
| 3'995,5 | | | | | | 4'845,0 |
| | | | 5'080,0 | | | |
| | | | 5'929,0 | | | |
| 7'132.5 | 7'500,0 | 7'121,1 | 7'412,0 | | | |
| | | | 7'945,0 | | | |
| | | | 8'481,1 | | | |
| 9'248,6 | | | | 9'201,2 | 9'762,5 | |
| | 11'102,8 | | 11'703,0 | | | |
| 14'186,0 | 14'142,2 | 14'217,1 | 14'489,0 | 14'192,4 | | |
| | | 14'620,2 | 14'647,0 | | | |
| | | | 15'887,0 | | | |
| 18'287,0 | | | | 18'367,4 | 17'086,6 | |
| 18'372,0 | | 18'461,6 | | | 19'441,4 | |
| | | 24'885,3 | | | | |
| | | 25'810,2 | | 25'281,1 | | |
| 28'331,5 | 28'283,0 | 28'660,0 | 27'783,0 | | | |
| | | | 30'391,0 | | | |
| 32'480,3 | | | | 32'573,6 | 33'971,7 | |
| | | | | 36'644,3 | | |

**Tabelle 2**

| Growth Factor Complex: HPLC-Retentionszeiten (in Minuten) | | | | | |
|---|---|---|---|---|---|
| Säule: Nucleosil C₁₈ | | | | | |
| Detektionswellenlänge: 275 nm | | | | | |
| Mobile Phase: Gradient mit Citronensäure 0,1 M und Acetonitril | | | | | |
| Proben: in 0.1 M Citronensäure gelöst (1 %-ige Lösung) | | | | | |

| GFC aus Kuhmilchpulver | GFC aus Kuhmilchmolke | GFC aus menschl. Milch | GFC aus Hühnereiern | GFC aus Fischrogen | Dialysewasser |
|---|---|---|---|---|---|
| 0,86 | | 0,87 | 0,84 | 0,85 | |
| 1,21 | 1,29 | 1,17 | 1,38 | 1,24 | 1,21 |
| 1,74 | 1,70 | 1,72 | 1,79 | 1,78 | 1,77 |
| | | | | | 1,94 |
| | | | | 2,21 | |
| 2,44 | | 2,63 | | 2,63 | 2,62 |
| 2,88 | | 2,93 | 2,86 | 2.91 | |
| 3,89 | | 3,86 | 3,85 | 3,93 | 3,91 |
| | | | | | 4,67 |
| | | 5,03 | | 5,04 | |
| 6,99 | 6,86 | | 6,93 | | |
| | | | | 7,36 | |
| | | 7,75 | | | |
| | | | | 8,54 | |
| | | 8,71 | | | 8,82 |
| | 9,17 | 9,28 | | | |
| 9,49 | 9,87 | | 9,55 | 9,31 | |
| | | | | 9,79 | |
| | | | | 9,82 | |
| 10,26 | | 10,02 | | | 10,10 |
| | | 10,53 | 10,45 | | |
| | | 11,79 | | | |
| | | | 13,07 | 12,93 | |
| | | 13,32 | 13,20 | | |
| | | | | 14,32 | |
| | | | 15,06 | | |
| | | 16,39 | | | 16,46 |
| 17,84 | | 17,83 | | | |
| 18,42 | | 18,50 | | 18,35 | 18,59 |
| 19,02 | | | | | |

Der erfindungsgemässe Komplex bestehend aus Wachstumsfaktoren enthält vorwiegend Lactose und ungefähr 2 % bis 15 % Proteine. Der Proteinanteil kann durch Dialyse stark erhöht werden. Der Gewichtsanteil an Proteinen setzt sich für die verschiedenen Growth Factor Complexes wie folgt zusammen (Bestimmungsmethode nach M.M. Bradford, Analytical Biochemistry, 1976, 72, 248-254):
- GFC aus Kuhmilchpulver: 5,0 Gew.-% Proteine
- GFC aus Kuhmilchpulver, dialysiert: 21,0 Gew.-% Proteine
- GFC aus Kuhmilchmolke: 5,9 Gew.-% Proteine
- GFC aus menschl. Milch: 14,5 Gew.-% Proteine
- GFC aus menschl. Milch, dialysiert 76,0 Gew.-% Proteine
- GFC aus Hühnereiern: 10,5 Gew.-% Proteine
- GFC aus Fischrogen: 2,5 Gew.-% Proteine

Bis anhin war das Verfahren zur Gewinnung von Wachstumsfaktoren sehr zeitund kostenaufwendig und mit einer sehr geringen Ausbeute an lediglich einzelnen Wachstumsfaktoren verbunden.

Gemäss Patentschrift EP 313 515 wird ein Verfahren zur Herstellung eines Milch-Wachstumsfaktors beschrieben, bei welchem das Ausgangsmaterial einer oder mehrerer chromatographischer Methoden und bei Bedarf weiteren Reinigungsprozessen unterworfen wird. Die Ausbeute aus diesem umfangreichen Herstellungsverfahren ergibt Wachstumsfaktoren aus Milch in 10³ bis über 10⁷ angereicherter Form, wobei jedoch ausgehend von 25-50 kg Milchpulver lediglich µg-Mengen Wachstumsfaktoren (MGF) erhalten werden. WO 95/29933 beschreibt die Herstellung mehrerer Wachstumsfaktoren aus Milch. Das sehr umständliche Verfahren benützt als Ausgangsmaterial eine pasteurisierte Molke, die vorgängig einer Mikrofiltration unterworfen wurde (Einzelheiten sind in der australischen Patentschrift No. 645589 beschneben). Danach wird das proteinhaltige Material an einem Kationenaustauscher adsorbiert, der mit 50 mM Natriumcitrat-Puffer behandelt worden war. Nach einem Waschvorgang wird das Protein eliminiert (mit 0,4 M NaCl, das zu 10 mM Natriumcitrat pH 6,5 gegeben wurde), gegen Wasser diafiltriert, mittels Ultrafiltration konzentriert und gefriergetrocknet. Weitere Reinigungsschritte liefern Fraktionen, die 10 mg Lactalbumin pro ml enthalten (die Fraktionen bestehen jeweils aus 100 µl). Patentschrift WO 95/26984 beinhaltet die Herstellung eines insulin-ähnlichen Wachstumsfaktors aus Kuhmilchmittels eines Kationen-Austauschers und anschliessender Dialyse. JP 6279312 beschreibt ebenfalls die Herstellung eines insulin-ähnlichen Wachstumsfaktors durch Erhitzen von Milch oder Molke auf eine spezifische Temperatur, Abzentrifugieren und Passieren des Überstandes durch einen Ultrafilter mit Fraktionen von 8 kDa.

Im Gegensatz zum Stand der Technik offenbart die vorliegende Erfindung ein vereinfachtes und kostengünstiges Verfahren mit einer hohen Ausbeute: Aus 1 kg handelsüblichem Milchpulver werden 350 g bis 420g Kohlenhydrate, in welchen der aktive Growth Factor Complex enthalten ist, isoliert. Dieser Komplex besteht - wie bereits vorstehend erwähnt - grösstenteils aus Milchzucker. Der Proteinanteil beträgt im Falle von Kuhmilch ungefähr 5 %. Durch Dialyse kann erwünschtenfalls der Milchzucker weitgehend entfernt werden. Für therapeutische Zwecke ist jedoch die Verdünnung der Faktoren mit Milchzucker vorteilhaft, da eine korrekte und reproduzierbare Dosierung der biologisch hochaktiven Faktoren so wesentlich einfacher gestaltet werden kann.

Die Wirkung von einzelnen Wachstumsfaktoren aus Milch wurde in den letzten 15 Jahren eingehend untersucht. So wurde unter anderem nachgewiesen, das s die in der Milch enthaltenen biologisch aktiven Polypeptide physiologische Funktionen übernehmen, wie z.B. die Regulation, Stimulation, aber auch Hemmung von verschiedenen Zellfunktionen (Am. J. Med. Sci., 1991, 301, 2, 124-132). Untersuchungen von Pittelkow (Advances in Dermatology, 1991, 7, 55-81) konnten die Wirkung einiger Milchpeptide als natürliche Mediatoren zellulärer Prozesse nachweisen, die bei der Aufrechterhaltung der Dermisstruktur eine wichtige Rolle spielen.

Die biologische Aktivität von Wachstumsfaktoren kann anhand des Migrationstests und/oder Proliferationstests in vitro ermittelt werden. Der Migrationstest nach Bürk (Proc. Nat. Acad. Sci., 1973, 70, 2, 369-372) erfolgt durch "Wundsetzung" in einer Monolayer-Zellkultur (z.B. Balb/C-3T3-Fibroblasten) Bei "verletzten" Zellkulturen, die nicht mit Wachstumsfaktoren behandelt werden, wandern die Zellen nicht über die Grenzlinie (1 cm breit) in die Wundfläche ein. Erst die Zugabe von Milch-Wachstumsfaktoren induzierte die Zellen, über die Grenzlinie in die Wundfläche und darüber hinaus zu wandern Auch der Proliferatronstest, ebenfalls an Balb/C-3T3-Fibroblasten durchgeführt, ergab bei Behandlung mit Milch-Wachstumsfaktoren eine Steigerung der Fibroblastenbildung

Der erfindungsgemässe Growth Factor Complex findet nach entsprechender Formulierung mit pharmazeutischen Hilfs- und Tragerstoffen in der Medizin vielseitige Verwendung, wie beispielsweise:
- zur Behandlung von Wunden, Verbrennungen, Hamatomen, Ekzemen, Geschwüren und dgl. mit beschleunigter Heilung
- zur Behandlung der Mundschleimhaut, we z B bei Parodontose, nach Zahnextraktionen und dgl. mit beschleunigter Heilung
- zur Behandlung der Nasenschleimhaut, wie z.B. bei trockener Nasenschleimhaut, bei Blutungsneigung, nach Operationen und dgl. mit beschleunigter Heilung
- zur Verbesserung der Dermisstruktur, wie z. B Narbenhetlung, Cellulitis, Altershaut und dgl.
- zur Behandlung und Vorbeugung von Alopecia
- zur Behandlung von Krebserkrankungen, wie z B. Hautkrebs, Lungenkrebs, Magenkrebs und dgl.
- zur Behandlung von gastrointestinalen Störungen, Krankheiten und Geschwüren
- zur Behandlung und Vorbeugung der Osteoporose
- als Nahrungssupplement, z.B. bei der Säuglingsernährung, bei parenteraler Ernährung und dgl.
- zur Ausbildung der Muskelmasse
- in der Veterinärmedizin zur Steigerung der Milchproduktion bei Säugetieren.

Im Stand der Technik ist bisher als Wirksubstanz allerdings nur ein einzelner oder die Kombination von zwei Wachstumsfaktoren beschrieben. WO 95/29933 beinhaltet einen Wachstumsfaktoren, der mit GFE-2 bezeichnet und aus Milch gewonnen wird. Dieser Wachstumsfaktor findet zur Wundbehandlung sowie bei gastrointestinalen Störungen, Krankheiten oder Geschwüren Verwendung. EP 367447 beschreibt die Verwendung des Human Cell Transforming Growth Factor zur Wachstumsstimulation von Epithelzellen, wie z.B. zur Behandlung von Wunden, Geschwüren, Verbrennungen oder zur Reepithelisation nach Radiotherapie. FR 2472385 und FR 2533438 beinhalten die kosmetische Verwendung von verschiedenen isolierten Wachstumsfaktoren. JP 6040858 verwendet den Fibroblast Growth Factor in einem Haartomkum zur Prävention und Behandlung von Alopecia. EP 313 515 beschreibt die Verwendung eines Milch-Wachstumsfaktors als Zahnpaste, Mundwasser, kosmetischen und Nahrungsmittelzubereitungen.

Ein weiterer Vorteil der vorliegenden Erfindung liegt in der verbesserten Wirksamkeit des erfindungsgemässen Growth Factor Complex, die offenbar aus einer Synergie der einzelnen im Komplex enthaltenen Wachstumsfaktoren resultiert. Wie in EP 313 515 geoffenbart, haben verschiedene Wachstumsfaktoren in Kombination gebracht eine synergistische Wirkung. In der genannten Patentschrift wird ein Milch-Wachstumsfaktor mit dem Epidermal Growth Factor (EGF) oder dem Transforming Growth Factor-α (TGF-α) kombiniert, um eine bessere Wirkung bei der Wundheilung zu erzielen. Die vorliegende Erfindung ist dahingehend verbessert, dass das Endprodukt bereits aus einer Kombination von verschiedenen Wachstumsfaktoren besteht, ohne dass vorausgehend ein einzelner Wachstumsfaktor isoliert und anschliessend mit einem anderen Faktoren kombiniert werden muss.

Der erfindungsgemässe Growth Factor Complex kann in alle für die genannten Indikationsgruppen üblichen galenischen Formen als Wirkstoff verarbeitet werden. Der Wirkstoffgehalt (Growth Factor Complex) in Arzneimittelzubereitungen beträgt 0,001 % bis 99 %, vorzugsweise 0,01 % bis 50 % und besonders bevorzugt 0,1 % bis 10 %. Ganz besonders bevorzugt ist für die topische Anwendung ein Wirkstoffgehalt von 0,5 % und für die orale Anwendung von 75 %.

Der Growth Factor Complex kann zu allen Arzneimittelformen, die zur lokalen Behandlung eingesetzt werden, verarbeitet werden. Solche Arzneimittelzubereitungen sind beispielsweise topisch applizierbare Präparate für Haut und Schleimhäuten wie zum Beispiel Salben, Crèmes, Gele, Pasten, wie auch Zahnpasten, Emulsionen, Lösungen auch in Form von Mundwasser, Sprays oder als Badezusatz, Pflaster und Gazen, die Salbe oder Paste enthalten

Der Growth Factor Complex kann ebenfalls zu parenteralen Arzneimittelzubereitungen, wie Infusions- und Injektionslösungen verarbeitet werden.

Orale Nahrungsmittelzubereitungen sind beispielsweise im Magensaft zerfallende Tabletten und Dragées oder andere feste Zubereitungen, sogenannte osmotische Pumpsysteme, ein- und mehrschichtige feste Zubereitungen, die eine verzögerte Freisetzung des Growth Factor Complex gewährleisten, Pellets in Kapseln oder gepresst mit sofortiger oder verzögerter Freisetzung des Growth Factor Complex, Lösungen der Substanzen in Weichgelatinekapseln oder nach speziellen Methoden versiegelten Hartgelatinekapseln oder anderen Umhüllungen, in.Wasser oder anderen Getränken lösliche Formen, wie zum Beispiel Brausetabletten, Brausegranulate, Lösungstabletten und Lösungsgranulate, flüssige Zubereitungen wie Tropfen oder Sirupe zur Einnahme als Konzentrat oder verdünnt in Wasser oder anderen Getränken.

Bei der Herstellung der pharmazeutischen Präparate kommen konventionelle Lösungs-, Lyophilisierungs-, Misch- und Suspendierverfahren zur Anwendung.

Zur Herstellung von topischen Arzneimittelzubereitungen eignen sich wässrige Lösungen des Growth Factor Complex, die entweder zu einer hydrophilen Salbengrundlage, z.B. Macrogol- bzw. Polyethylenglykolsalbe, zu Crèmes, die hydrophobe Lipidbestandteile, Wasser und Tenside enthalten, zu Gelen mit hydrophilen makromolekularen Verbindungen, wie z.B. Gelatine und Celluloseether, zu Pasten aus einer hochkonzentrierten Suspensionssalbe, zu Emulsionen, unter Verwendung von Öl, arabischem Gummi und Wasser, verarbeitet werden. Die Herstellung der topischen Arzneimittel erfolgt nach konventionellen Methoden Zur parenteralen Verabreichung eignen sich in erster Linie wässrige Lösungen, ferner Suspensionen des Wirkstoffs, wie entsprechende ölige Injektionssuspensionen, wobei man geeignete lipophile Lösungsmittel oder Vehikel, wie pflanzliche Öle, zum Beispiel Sesamöl, oder Fettsäureester, zum Beispiel Ethyloleat oder Triglyceride, verwendet, oder wässrige Injektionssuspensionen, welche viskostätserhöhende Stoffe, zum Beispiel Natrium-Carboxymethylcellulose, Sorbit und/oder Dextran und gegebenenfalls auch Stabilisatoren enthalten.

Nahrungsmittelzubereitungen zur oralen Anwendung werden beispielsweise erhalten, indem man den Growth Factor Complex mit festen Trägerstoffen kombiniert, das erhaltene Gemisch gegebenenfalls granuliert, und das Gemisch oder Granulat, falls gewünscht oder notwendig, nach Zugabe geeigneter Hilfsstoffe zu Tabletten oder Dragee-Kernen verarbeitet. Geeignete Trägerstoffe sind insbesondere Füllstoffe, wie Zucker, zum Beispiel Lactose, Saccharose, Mannit oder Sorbit, Cellulosepräparate und/oder Calciumphosphate, zum Beispiel Tricalciumphosphat oder Calciumhydrogenphosphat, ferner Bindemittel, wie Stärkekleister unter Verwendung von zum Beispiel Mais-, Weizen-, Reis- oder Kartoffelstärke. Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alinsäure oder ein Salz davon, wie Natriumalginat. Hilfsmittel sind in erster Linie Fliessregulier- und Schmiermittel, zum Beispiel Kieselsäure, Talk, Stearinsäure, oder Salze davon, wie Magnesium- oder Calciumstearat, und/oder Polyethylenglykol.

Dragée-Kerne werden mit geeigneten, gegebenenfalls magensaftresistenten Überzügen versehen, wobei man unter anderem konzentrierte Zuckerlösungen, welche gegebenenfalls arabischen Gummi, Talk, Polyvinylpyrrolidon, Polyethylenglykol und/ oder Titandioxid enthalten, Lacklösungen in geeigneten organischen Lösungsmitteln oder Lösungsmittelgemischen oder, zur Herstellung von magensaftresistenten Überzügen, Lösungen von geeigneten Cellulosepräparaten, wie Acetylcellulosephthalat oder Hydroxypropylmethylcellulosephthalat, verwendet.

Weitere, oral anwendbare Nahrungsmittelzubereitungen sind Steckkapseln aus Gelatine, sowie weiche, geschlossene Kapseln aus Gelatine und einem Weichmacher, wie Glycerin oder Sorbitol. Die Steckkapseln können den Growth Factor Complex in Form eines Granulates, zum Beispiel im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Growth Factor Complex vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Die vorliegende Erfindung wird durch die nachfolgenden Beispiele verdeutlicht:

### Beispiel 1: Herstellung von Kuhmilchpulver-GFC

| | |
|---|---|
| Kuhmilchpulver, entfettet | 150 g |
| Wasser, entionisiert | 700 g |
| Milchsäure (80 %-ig) | 120 g |
| Aceton | 3'560 g |
| Wasser, entionisiert | 300 g |
| Aceton | 150 g |

1. 150 g entfettetes Kuhmilchpulver langsam (innerhalb von 5 Minuten) unter Rühren (Heidolph-Rührmotor, ca. 200 UpM) in ein Becherglas in 700 g entionisiertes Wasser eintragen. Die Suspension während 20 Minuten weiterrühren.
2. Anschliessend 120 g Milchsäure langsam (innerhalb von 5 Minuten) zu der Suspension giessen und während 15 Minuten weiterrühren.
3. Den Becherglasinhalt in Zentrifugiergefässe überführen und während 20 Minuten bei 6'000 UpM abzentrifugieren.
4. Die überstehende Flüssigkeit abgiessen. Den festen Rückstand in den Gefässen mit ca. 300 g entionisiertem Wasser anteigen und nochmals abzentrifugieren.
5. Die überstehende Flüssigkeit zusammen mit der vorherigen durch einen Saftfilter filtrieren. Das Filtrat ist hellgelb und opaleszent
6. 900 g Filtrat unter Rührung (Heidolph-Rührmotor, 200 UpM) in einem Erlenmeyerkolben langsam (innerhalb 5 Minuten) mit 3'560 g Aceton versetzen und 5 Minuten nachrühren. Den erhaltenen Niederschlag über Nacht bei Raumtemperatur stehen lassen.
7. Den Niederschlag am nächsten Tag über eine Glassinternutsche G4 abnutschen, dreimal mit ca. je 50 g Aceton nachwaschen und bei 40°C im Vakuumtrockenschrank trocknen, vorteilhaft unter Stickstoffbegasung.
   Ausbeute: ca. 58 g Kuhmilchpulver-GFC.

### Beispiel 2: Herstellung von Muttermilch-GFC

| | |
|---|---|
| menschliche Milch | 600 ml |
| Milchsäure (80 %-ig) | 90 ml |
| Aceton | 1'500 ml |

1. 600 ml menschliche Milch während 20 Min. bei 6'000 UpM zentrifugieren. Danach obenaufschwimmendes Fett abtrennen und die restliche Lösung im Vakuum auf 300 ml einengen.
2. Anschliessend 90 ml Milchsäure langsam (innerhalb von 5 Minuten) zu der Lösung giessen und während 15 Minuten weiterrühren.
   Fortfahren entsprechend Beispiel 1, Punkt 3 bis 7.
   Ausbeute: ca. 40 g Muttermilch-GFC.

### Beispiel 3: Herstellung von Hühnereier-GFC

| | |
|---|---|
| Eigelb | 6 |
| Wasser, entionisiert | 200 ml |
| Milchsäure (80 %-ig) | 100 ml |
| Aceton | 1'500 ml |

1 6 Eigelbe im Vakuum zur Trockene eindampfen (ca 44 g Trockensubstanz) und in 200 ml entionisiertem Wasser aufnehmen.
2. Anschliessend 100 ml Milchsäure langsam (innerhalb von 5 Minuten) zu der Suspension giessen Es entsteht eine sehr feine, nicht filtrierbare Ausfällung
3 Die Suspension in einem Dialyseschlauch (Cutoff: 3'000 MG) gegen Wasser während 2 Tagen dialysieren.
4. 300 ml Lösung aus dem Dialyseschlauch unter Rührung (Heidolph-Rührmotor, 200 UpM) in einem Erlenmeyerkolben langsam (innerhalb 5 Minuten) mit 1'500 ml Aceton ausfällen und 5 Minuten nachrühren. Die Fällung über Nacht bei Raumtemperatur stehen lassen.
5. Den Niederschlag am nächsten Tag über eine Glassinternutsche G4 abnutschen, dreimal mit ca. je 50 ml Aceton nachwaschen und bei 40°C im Vakuumtrockenschrank trocknen.
   Ausbeute: ca. 4 g Hühnereier-GFC.

### Beispiel 4: Herstellung von Fischrogen-GFC

| | |
|---|---|
| Forelleneier | 100 g |
| Wasser, entionisiert | 500 ml |
| Milchsäure (80 %-ig) | 80 ml |
| Aceton | 2'500 ml |

1. 100 g Forelleneier zusammen mit 500 ml entionisiertem Wasser im Mixer zerkl einem.
2. Anschliessend 80 ml Milchsäure langsam (innerhalb von 5 Minuten) zu der Suspension giessen und während 15 Minuten weiterrühren.
3. Die Fällung in Zentrifugiergefässe geben und während 20 Minuten bei 6'000 UpM abzentrifugieren. Die überstehende Flüssigkeit (ca. 500 ml) abgiessen.
4. 500 ml Filtrat unter Rührung (Heidolph-Rührmotor, 200 UpM) in einem Erlenmeyerkolben langsam (innerhalb 5 Minuten) mit 2'500 ml Aceton ausfällen und 5 Minuten nachrühren. Die Fällung über Nacht bei Raumtemperatur stehen lassen.
5. Den Niederschlag am nächsten Tag über eine Glassinternutsche G4 abnutschen, dreimal mit ca je 50 ml Aceton nachwaschen und bei 40°C im Vakuumtrockenschrank trocknen.
   Ausbeute: ca 1,5 g Fischrogen-GFC.

### Beispiel 5: Herstellung von Menschblut-GFC

| | |
|---|---|
| menschliches Blut | 150 ml |
| Wasser, entionisiert | 225 ml |
| Milchsäure (80 %-ig) | 45 ml |
| Aceton | 1'600 ml |

1. 150 ml menschliches Blut mit 225 ml entionisiertem Wasser verdünnen.
2. Anschliessend unter Rühren (Heidolph-Rührmotor, ca. 200 UpM) 45 ml Milchsäure langsam (innerhalb von 5 Minuten) zu der Suspension giessen und während 15 Minuten weiterrühren.
3. Die Fällung in Zentrifugiergefässe überführen und während 20 Minuten bei 6'000 UpM abzentrifugieren. Die überstehende Flüssigkeit (ca. 320 ml) abgiessen.
4. 320 ml Filtrat unter Rührung (Heidolph-Rührmotor, 200 UpM) in einem Erlenmeyerkolben langsam (innerhalb 5 Minuten) mit 1'600 ml Aceton ausfällen und 5 Minuten nachrühren. Die Fällung über Nacht bei Raumtemperatur stehen lassen.
5. Den Niederschlag am nächsten Tag über eine Glassintemutsche G4 abnutschen, dreimal mit ca. je 50 ml Aceton nachwaschen und bei 40°C im Vakuumtrockenschrank trocknen.
   Ausbeute: ca. 22 g Menschenblut-GFC.

### Beispiel 6: Herstellung von Kuhmilchmolke-GFC

| | |
|---|---|
| Kuhmilchmolkepulver | 100 g |
| Wasser, entionisiert | 500 ml |
| Milchsäure | 100 ml |
| Aceton | ca. 3'500 ml |

1. 100 g Molkepulver unter Rühren (Heidolph-Rührmotor, ca. 200 UpM) in 500 ml Wasser anschwemmen.
2. Anschliessend mit 100 ml Milchsäure versetzen und unter den gleichen Bedingungen ca. 1 Stunde weiterrühren.
3. Die Fällung in Zentrifugiergefässe überführen und während 20 Minuten bei 5'500 UpM abzentrifugieren. Die überstehende Flüssigkeit (ca. 570 ml) abgiessen.
4. Das Filtrat in zwei Teilen mit je 1'500 ml Aceton unter Rühren (Heidolph-Rührmotor, 200 UpM) ausfällen. Die Fällung über Nacht bei Raumtemperatur stehen lassen.
5. Den Niederschlag am nächsten Tag über eine Glassinternutsche G4 abnutschen, dreimal mit ca. 50 ml Aceton nachwaschen und bei 40°C im Vakuumtrockenschrank trocknen.
   Ausbeute: ca. 73,4 g Kuhmilchmolke-GFC

### Beispiel 7: GFC-Wundgel 0,5 %

| 100 g Wundgel enthalten: | |
|---|---|
| Carbopol 980 NF | 0,50 g |
| Wasser, entmineralisiert | 92,84 g |
| Tris(-hydroxymethyl-)aminomethan 1 M in H20 | 6,16 g |
| Growth Factor Complex (GFC) | 0,50 g |

1. 60 g entmineralisiertes Wasser in ein 200 g-Becherglas einwiegen und auf dem Magnetrührer bei mittlerer Geschwindigkeit rühren.
2. 0,5 g Carbopol 980 NF langsam dem Wasser unter Rühren beigeben, 10 Min. weiterrühren und danach 15 Min. quellen lassen.
3. In einem Reagenzglas 5 g Tris(-hydroxymethyl-)aminomethan einwiegen, 0,5 g GFC zufügen, gut suspendieren.
4. Die GFC-Lösung zur Gelmasse geben und mit einem Spatel gut vermischen.
5. Unter Rühren den pH-Wert mit Tris(-hydiroxymethyl-)aminomethan 1 M auf 6-7 einstellen.
6. Unter Rühren die Gelmasse mit deminer alisiertem Wasser auf 100 g auffüllen und etwa 5 Min. weiterrühren.

### Beispiel 8: GFC-Zahnpaste 0,5 %

| 100 g Zahnpaste enthalten: | |
|---|---|
| Growth Factor Complex (GFC) | 0,50 g |
| Calciumcarbonat | 37,00 g |
| Aerosil 200 | 1,00 g |
| Glycerin | 25,00 g |
| Paraffin | 0,50 g |
| Pfefferminzöl | 1,00 g |
| Na-Fluorid | 0,76 g |
| Saccharin | 0,01 g |
| Wasser, entmineralisiert | 27,28 g |
| Carboxymethylcellulose | 0,80 g |
| Na-Laurylsulfat | 2,00 g |
| Wasser entmineralisiert | 4,15 g |

1. GFC, Calciumcarbonat und Aerosil 200 in eine Reibschale geben und mit dem Pistill gut mikronisieren.
2 Glycerin, Paraffin und Pefferminzöl zugeben und mit dem Pistill anteigen.
3. Demineralisiertes Wasser in ein Becherglas geben, Na-Fluorid und Saccharin zugeben und mit dem Magnetrührer während etwa 5 Min. rühren und zum angeteigten Calciumcarbonat geben.
4 Na-Laurylsulfat in ein Becherglas geben und das restliche Wasser zufügen. Auf ca. 30°C erwärmen, unter ständigem Rühren suspendieren und ebenfalls zum angeteigten Calciumcarbonat geben.
5. Die Masse gut von Hand verrühren und anschliessend über die 3-Walzen-Mühle bei Stufe 3 geben und nochmals verrühren.

Der pH-Wert der erhaltenen Paste liegt bei 10. Sofern aus organoleptischen Gründen erwünscht, kann der pH-Wert mit Citronensäure auf einen neutralen oder leicht sauren Bereich eingestellt werden.

### Beispiel 9 GFC-Injektionslösung 2 %

| Zusammensetzung für 1 Liter Injektionslösung: | |
|---|---|
| Growth Factor Complex | 20,0 g |
| Ascorbinsäure | 45,0 g |
| Wasser zu Injektionszwecken | 935,0 g |

1 935 g Wasser zu Injektionszwecken in einen Ertenmeyerkolben einwiegen
2. 45 g Ascorbinsäure zugeben und bei geschlossenem Kolben mit dem Magnetrührer bei 900 UpM in ca. 10 Min. zur Lösung bringen.
3. 20 g GFC zufügen und etwa eine Stunde wie oben weiterrühren, bis der GFC fast gelöst ist.
4. Losung durch Sterilfilter direkt in eine sterile Braunglasflasche filtrieren.

### Beispiel 10: GFC-Pulver zur Injektion 100 mg

| Zusammensetzung für 200 Fläschchen, enthaltend jeweils 100 mg GFC: | |
|---|---|
| Growth F actor Complex | 20,0 g |

| Zusammensetzung für 200 Solvensampullen, enthaltend jeweils 5 ml Losung: | |
|---|---|
| Ascorbinsäure | 45,0 g |
| Wasser zu Injektionszwecken | 935,0 g |

1. 100 mg GFC in sterile Fläschchen abfüllen.
2. 935 g Wasser zu Injektionszwecken in einen Erlenmeyerkolben einwiegen.
3. 45 g Ascorbinsäure zum Wasser geben und bei geschlossenem Kolben mit dem Magnetrührer bei 900 UpM in ca. 10 Min. zur Lösung bringen.
4. Lösung durch Sterilfilter direkt in eine sterile Braunglasflasche filtrieren und anschliessend jeweil 5 ml der Lösung in Solvensampullen geben.
5. Bei Gebrauch den Inhalt einer Solvensampulle in ein steriles Fläschchen mittels Injektionsspritze geben und den GFC durch sorgfältiges Schütteln des Fläschchens in Lösung bringen.

### Beispiel 11: GFC-Tabletten 500 mg

| 1000 Tabletten enthalten: | |
|---|---|
| Growth Factor Complex | 500,0 g |
| Cellulose, mikrokristalline | 86,0 g |
| Na-Carboxymethylstärke | 50,6 g |
| Povidone K30 | 30,0 g |
| Kieselsäure, mikronisiert, porös, synth. | 3,2 g |
| Rizinusöl, gehärtet | 5,2 g |
| Isopropanol | zum Befeuchten |

1. GFC, Cellulose, Na-Carboxymethytstärke und Povidone abwiegen und mit Sieb (Maschenweite 710 µm) sieben respektive mischen.
2. Die Mischung mit Pistill in der Reibschale gut verreiben und mit ca. 180 g Isopropanol befeuchten.
3 Die feuchte Mischung während 3 Stunden bei 50°C im Trockenschrank trocknen und anschliessend durch Sieb (Maschenweite 710 µm) geben.
4. Kieselsäure und Rizinusöl zum Granulat geben und in einem verschlossenen Behälter gut mischen.
5. Auf der Tablettenpresse (Korsch EK0) mit Stempel von 13 mm Durchmesser Tabletten mit einem Gewicht von je 675 mg (entsprechend 500 mg GFC) pressen.

## Patentansprüche

1. Verfahren zur Gewinnung eines Wachstumsfaktoren-Komplexes (Growth Factor Complex), aus natürlichem und Wachstumsfaktoren-enthaltendem Material, **dadurch gekennzeichnet, dass** man
a) das Ausgangsmaterial mit Wasser versetzt,
b) den pH-Wert durch Zugabe von Säure auf 2,5 bis 3,2 einstellt,
c) die entstandene Fällung von Überstand abtrennt,
d) die erhaltene Wachstumsfaktoren-enthaltende wässrige Lösung mit einem mit Wasser mischbaren organischen Lösungsmittel, ausgewählt aus niederen Alkanolen oder niederen Dialkylketonen, versetzt,
e) den erhaltenen Niederschlag abtrennt und trocknet und
f) gewünschtenfalls das erhaltene Produkt dialysiert.

2. Verfahren nach Anspruch 1, **dadurch gekennzeichnet, dass** man als natürliches und Wachstumsfaktoren-enthaltendes Material Milch von Säugetieren sowie die entsprechende Molke, Vogeleier, Fischrogen, Blut von Säugetieren, Urin von Säugetieren sowie Bienenhonig und pflanzliche Samen verwendet.

3. Verfahren nach Anspruch 1, **dadurch gekennzeichnet, dass** man als Säure zur Einstellung des pH-Wertes von 2,5 bis 3,2 eine nicht-oxidierende Mineralsäure oder eine Mono-, Di- oder Tricarbonsäure oder eine von einer solchen Mono-, Di- oder Tricarbonsäure abgeleitete Hydroxycarbonsäure verwendet.

4. Verfahren nach Anspruch 1, **dadurch gekennzeichnet, dass** man als mit Wasser mischbares organisches Lösungsmittel Methanol, Ethanol, n-Propanol, Isopropanol, Aceton oder Diethylketon verwendet.

5. Verfahren nach Anspruch 1, **dadurch gekennzeichnet, dass** man zur Abscheidung der Wachstumsfaktoren ein mit Wasser mischbares organisches Lösungsmittel mit der Wachstumsfaktoren-enthaltenden wässrigen Lösung in einem Volumenverhältnis von 2 : 1 bis 10 : 1 versetzt.

6. Verfahren nach Anspruch **1 , dadurch gekennzeichnet, dass** man den erhaltenen Komplex aus Wachstumsfaktoren (nach dem Trocknen) mit einem fettlösenden Lösungsmittel extrahiert.

7. Komplex aus Wachstumsfaktoren, erhältlich dadurch, dass man
a) Wachstumsfaktoren-enthaltendes Material mit Wasser versetzt,
b) den pH-Wert durch Zugabe von Säure auf 2,5 bis 3,2 einstellt,
c) die entstandene Fällung vom Überstand abtrennt,
d) die erhaltene Wachstumsfaktoren-enthaltende wässrige Lösung mit einem mit Wasser mischbaren organischen Lösungsmittel, ausgewählt aus niederen Alkanolen oder niederen Dialkylketonen, versetzt,
e) den erhaltenen Niederschlag abtrennt und trocknet und
f) gewünschtenfalls das erhaltene Produkt dialysiert.

8. Arzneimittelzubereitung, **dadurch gekennzeichnet, dass** sie neben pharmazeutisch verträglichen Hilfs- und Trägerstoffen den Komplex aus Wachstumsfaktoren gemäss Anspruch 7 als aktive Komponente enthält.

9. Wachstumsfaktoren-enthaltende wässrige Lösung, erhältlich dadurch, dass man
a) Wachstumsfaktoren-enthaltendes Material mit Wasser versetzt,
b) den pH-Wert durch Zugabe von Säure auf 2,5 bis 3,2 einstellt und
c) die entstandene Fällung vom Überstand abtrennt.

## Claims

1. Process for the extraction of a Growth Factor Complex from natural and growth factors containing material, which is **characterized in that**
a) water is added to the starting material
b) the pH value is adjusted to 2.5 to 3.2 by the addition of acid,
c) the resulting precipitate is separated from the supernatant,
d) an organic solvent miscible with water, selected from lower alkanols or from lower dialkyl ketones, is added to the growth factors containing aqueous solution,
e) the resulting precipitate is separated off and dried, and
f) if desired, the product obtained is dialyzed.

2. Process according to claim 1, **characterized in that** natural and growth factors containing materials may be milk from mammals as well as the corresponding whey, bird's eggs, fish roe, blood from mammals, urine from mammals, as well as bee's honey and plant seeds.

3. Process according to claim 1, **characterized in that** a non-oxidizing mineral acid or a mono-, di- or tricarboxylic acid or a hydroxid carboxylic acid derived from such mono-, di- or tricarboxylic acid is used for adjusting the pH value from 2.5 to 3.2.

4. Process according to claim 1, **characterized in that** methanol, ethanol, n-propanol, isopropanol, acetone or diethyl ketone are used as organic solvent miscible with water.

5. Process according to claim 1, **characterized in that** the organic solvent miscible with water and the growth factors containing aqueous solution are mixed at a volume ratio of 2 : 1 to 10 : 1 for separating the Growth Factor Complex.

6. Process according to claims 1, **characterized in that** the Growth Factor Complex obtained after drying is extracted with a fat soluble solvent.

7. Growth Factor Complex obtainable by
a) adding water to growth factors containing material,
b) adjusting the pH value to 2.5 to 3.2 by the addition of acid,
c) separating the resulting precipitate from the supernatant,
d) adding an organic solvent miscible with water, selected from lower alkanols or from lower dialkyl ketones, to the growth factors containing aqueous solution,
e) separating off and drying the resulting precipitate, and
f) if desired, dialyzing the product obtained.

8. Pharmaceutical compositions, **characterized in that** the Growth Factor Complex according to claim 7 is contained as the active component in physiologically well tolerated auxiliary and carrier materials.

9. The growth factors containing aqueous solution obtainable by
a) adding water to growth factors containing material,
b) adjusting the pH value to 2.5 to 3.2 by the addition of acid, and
c) separating the resulting precipitate from the supernatant.

## Revendications

1. Procédé pour la production d'un complexe de facteurs de croissance (Growth Factor Complex), à partir de matières naturelles et contenant des facteurs de croissance, **caractérisé en ce que** l'on
a) mélange la matière de départ avec de l'eau,
b) ajuste le pH à une valeur entre 2,5 et 3,2 en ajoutant de l'acide,
c) sépare le précipité du surnageant,
d) mélange la solution aqueuse ainsi obtenue, contenant des facteurs de croissance, avec un solvant organique miscible à l'eau, choisi parmi les alcanols inférieurs ou les dialkylcétones inférieures,
e) sépare le précipité ainsi obtenu et le sèche, et
f) dialyse le produit obtenu, si on le souhaite.

2. Procédé conforme à la revendication 1, **caractérisé en ce que** l'on utilise, en tant que matière naturelle et contenant des facteurs de croissance, du lait de mammifère, ainsi que son petit-lait, des oeufs d'oiseau, des oeufs de poisson, du sang de mammifère, de l'urine de mammifère, ainsi que du miel d'abeilles et des semences végétales.

3. Procédé conforme à la revendication 1, **caractérisé en ce que** l'on utilise, en tant qu'acide pour ajuster le pH à une valeur entre 2,5 et 3,2, un acide minéral non oxydant ou un acide mono-, di- ou tricarboxylique ou un acide hydroxycarboxylique dérivé d'un tel acide mono-, di- ou tricarboxylique.

4. Procédé conforme à la revendication 1, **caractérisé en ce que** l'on utilise, en tant que solvant organique miscible à l'eau, du méthanol, de l'éthanol, du n-propanol, de l'isopropanol, de l'acétone ou de la diéthylcétone.

5. Procédé conforme à la revendication 1, **caractérisé en ce que**, dans le but de séparer les facteurs de croissance, l'on mélange un solvant organique miscible à l'eau avec la solution aqueuse contenant les facteurs de croissance, dans un rapport de volume se situant entre 2 : 1 et 10 : 1.

6. Procédé conforme à la revendication 1, **caractérisé en ce que** l'on extrait le complexe obtenu à partir de facteurs de croissance (après séchage) au moyen d'un solvant de graisse.

7. Complexe de facteurs de croissance obtenu par un procédé **caractérisé par le fait que** l'on
a) mélange une matière contenant des facteurs de croissance avec de l'eau,
b) ajuste le pH à une valeur entre 2,5 bis 3,2 en ajoutant de l'acide,
c) sépare le précipité du surnageant,
d) mélange la solution aqueuse ainsi obtenue, contenant des facteurs de croissance, avec un solvant organique miscible à l'eau, choisi parmi les alcanols inférieurs ou les dialkylcétones inférieures,
e) sépare le précipité ainsi obtenu et le sèche, et
f) dialyse le produit obtenu, si on le souhaite.

8. Préparation pharmaceutique **caractérisée en ce qu'**elle contient, outre des substances auxiliaires et des substances porteuses physiologiquement bien tolérées, le complexe de facteurs de croissance, conforme à la revendication 7, en tant que composante active.

9. Solution aqueuse contenant des facteurs de croissance, obtenue au moyen d'un procédé **caractérisé par le fait que** l'on
a) mélange une matière contenant des facteurs de croissance avec de l'eau,
b) ajuste le pH à une valeur entre 2,5 et 3,2 en ajoutant de l'acide,
c) sépare le précipité du surnageant.
